(19) Europäisches Patentamt
European Patent Office
Office européen des brevets

(11) Publication number: **0 200 968**
**B1**

(12) **EUROPEAN PATENT SPECIFICATION**

(45) Date of publication of patent specification: **29.08.90**

(51) Int. Cl.[5]: **C 07 D 401/12,**
C 07 D 403/12,
C 07 D 209/76, A 61 K 31/40

(21) Application number: **86105299.1**

(22) Date of filing: **17.04.86**

(54) **Succinimide derivatives, their production and use.**

(30) Priority: **17.04.85 JP 83297/85**

(43) Date of publication of application:
**12.11.86 Bulletin 86/46**

(45) Publication of the grant of the patent:
**29.08.90 Bulletin 90/35**

(84) Designated Contracting States:
**AT BE CH DE FR GB IT LI NL SE**

(56) References cited:
**EP-A-0 082 402**
**EP-A-0 109 562**
**EP-A-0 111 226**
**FR-A-2 506 771**
**GB-A-2 146 333**
**US-A-2 672 460**
**US-A-4 562 255**

**JOURNAL OF MEDICINAL CHEMISTRY, vol. 26, 1983, pages 194-203, American Chemical Society, US; J.P. YEVICH et al.: "Buspirone analogues. 1. Structure-activity relationships in a series of N-aryl- and heteroarylpiperazine derivatives"**

**The file contains technical information submitted after the application was filed and not included in this specification**

(73) Proprietor: **SUMITOMO PHARMACEUTICALS COMPANY, LIMITED**
**2-8, Doshomachi 2-chome, Chuo-ku**
**Osaka-shi Osaka-fu (JP)**

(72) Inventor: **Ishizumi, Kikuo**
**16-10, Uenonishi 2-chome**
**Toyonaka-shi Osaka-fu (JP)**
Inventor: **Antoku, Fujio**
**14-7, Mefu 2-chome**
**Takarazuka-shi Hyogo-ken (JP)**
Inventor: **Maruyama, Isamu**
**15-12, Nakanoosa Higashiuneno**
**Kawanishi-shi Hyogo-ken (JP)**
Inventor: **Kojima, Atsuyuki**
**21-4, Izumi-cho**
**Takarazuka-shi Hyogo-ken (JP)**

(74) Representative: **Vossius & Partner**
**Siebertstrasse 4 P.O. Box 86 07 67**
**D-8000 München 86 (DE)**

**EP 0 200 968 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

The present invention relates to novel succinimide derivatives, and their production and use. More particularly, it relates to novel succinimide derivates having a piperazinylbutyl group at the N-position or their acid addition salts, their preparation process and use as anti-anxiety drug.

EP—A—109 562 discloses succinimide derivatives of the formula

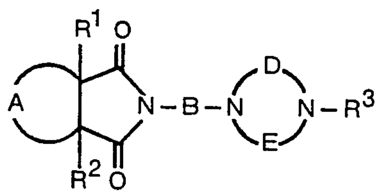

wherein A is straight or branched $C_2$—$C_6$ alkylene or alkenylene, B is straight or branched $C_3$—$C_5$ alkylene. D is straight or branched $C_2$—$C_3$ alkylene, E is straight or branched $C_2$—$C_3$ alkylene, $R^1$ and $R^2$ are each hydrogen or $C_1$—$C_4$ alkyl, or they may form a single bond and $R_3$ is a phenyl group optionally substituted with $C_1$—$C_4$ alkyl, $C_1$—$C_4$ alkoxy, halogen and or trifluoromethyl, a 2-pyridyl group optionally substituted with halogen, a 2-pyrimidyl group optionally substituted with halogen, a group of the formula:

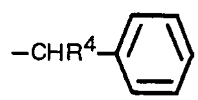

(in which $R^4$ is hydrogen or phenyl), a group of the formula:

CO—$R^5$ (in which $R^5$ is adamantlyl or furyl) or hydroxy ($C_2$—$C_4$)alkyl, and an acid addition salt thereof, which are useful as anti-anxiety drugs and/or anti-allergic drugs.

EP—A—82 402 describes succinimide derivatives representable by the following formula:

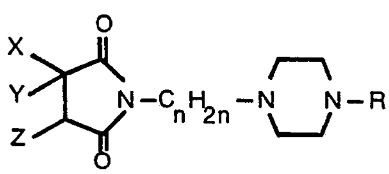

(I)

wherein X and Y are combined to form a group of the formula:

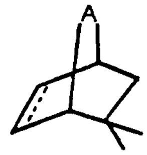

(wherein A is an oxygen atom, a methylene group or an ethylene group and a full line accompanying a broken line (------) is a single bond or a double bond) and Z is a hydrogen atom or, X and Z are combined to form a group of the formula:

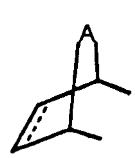

(wherein A and a full line are each as defined above) and Y is a hydrogen atom, R is a phenyl group, optionally subsituted with halogen, $C_1$—$C_4$ alkyl. $C_1$—$C_4$ alkoxy or trifluoromethyl, a 2-pyridyl group or a 2-pyrimidinyl group and n is an integer of 3 or 4, and pharmaceutically acceptable acid addition salts thereof, which are useful as antianxous substances.

J.P. Yerich et al. diclose in J. Med. Chem. Vol. 26 (1983), pp. 194—203 a group of substituted cyclic imides having anxiolytic activity including a compound of the formula:

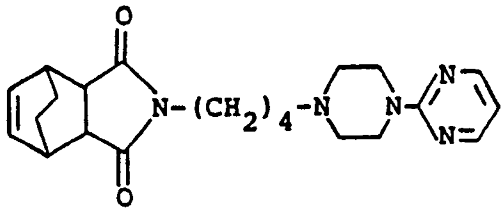

The succinimide derivatives of this invention can be represented by the formula:

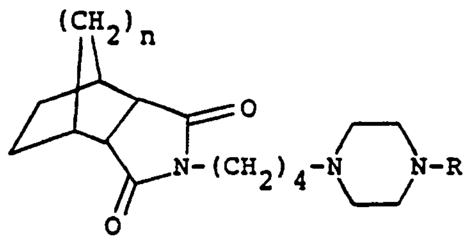

(I)

wherein R is a pyridyl or pyrimidinyl group each being substituted at least one substituent selected from the group consisting of halogen, lower alkyl, lower alkoxy, cyano, benzyloxy, hydroxyl and amino and n is an integer of 1 or 2.

In the above meanings, the term "halogen" includes fluorine, chlorine, bromine and iodine. The term "lower alkyl" includes a straight or branched alkyl group having 1 to 4 carbon atoms such as methyl, ethyl, n-propyl, isopropyl, n-butyl or isobutyl. The term "lower alkoxy" includes a straight or branched alkoxy group having 1 to 4 carbon atoms such as methoxy, ethoxy, n-propoxy, isopropoxy, n-butoxy or isobutoxy.

Preferred succinimide derivatives (I) are those wherein R is pyridyl group substituted by at least one substituent selected from the group consisting of halogen, methoxy and cyano or a pyrimidinyl group substituted by at least one substituent selected from the group consisting of halogen, methyl, methoxy, benzyloxy, hydroxyl and amino.

The succinimide derivatives (I) can form salts with acids. Suitable pharmaceutically acceptable acid addition salts are those with inorganic acids such as hydrochloric acid, hydrobromic acid, sulfuric acid and phosphoric acid or organic acids such as acetic acid, propionic acid, butyric acid, oxalic acid, succinic acid tartaric acid, citric acid, maleic acid and fumaric acid.

The succinimide derivatives (I) of the invention can be prepared by the process shown in the following reaction scheme:

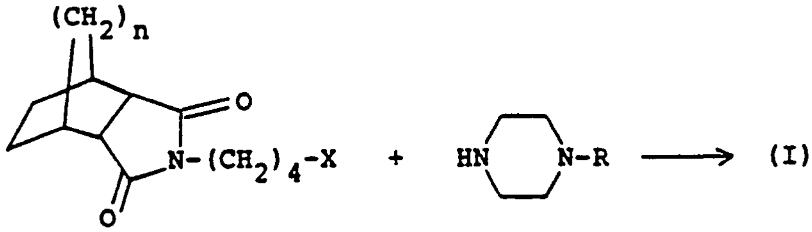

(II) (III)

wherein R and n are each as defined above and X is a halogen atom (e.g. chlorine, bromine, iodine).

The succinimide derivative (I) is obtainable by reacting compound (II) with compound (III), preferably in the presence of an acid binding agent, in an inert solvent (e.g. benzene, toluene, xylene, N,N-dimethylformamide, acetonitrile, n-butanol, acetone) at room temperature or elevated temperature. The acid binding agent may be chosen from alkali metal or alkaline earth metal carbonates, bicarbonates and hydrides (e.g. potassium carbonate, sodium bicarbonate, sodium hydride), organic tertiary amines (e.g. triethylamine, pyridine), etc.

Among the succinimide derivatives (I), the one of the following formula:

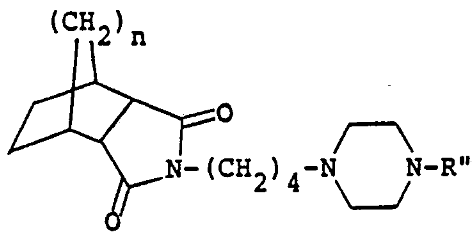

(I-b)

wherein R'' is a pyridyl or pyrimidinyl group substituted by hydroxy and n is as defined above, may be produced by subjecting a compound of the formula:

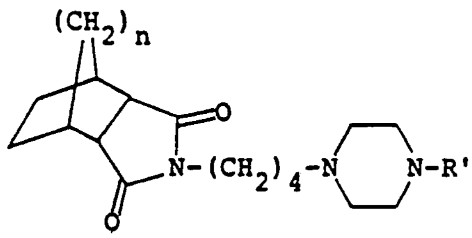

(I-a)

wherein R' is a pyridyl or pyrimidinyl group substituted by benzyloxy and n is as defined above, to reduction.

The above reduction is usually carried out by hydrogenating compound (I-a) in an inert solvent in the presence of a catalyst. Such a catalyst may be any catalyst conventionally used for hydrogenation. Specific examples are platinum catalysts (e.g. platinum black, platinum dioxide, platinum colloid), palladium catalysts (e.g. palladium black, palladium on carbon, palladium colloid), rhodium catalysts, nickel catalysts (e.g. Raney nickel, nickel oxide), etc. Examples of the solvent are lower alkanols (e.g. methanol, ethanol, isopropanol), water, acetic acid, ethyl acetate, tetrahydrofuran, dioxane, etc. Hydrogenation may be effected under atmospheric pressure or elevated pressure and at normal temperature or elevated temperature.

The starting compounds (II) and (III) used in the above process are known per se or can be produced from the known compounds by a conventional procedure. For instance, compound (II), i.e. the N-halobutylsuccinimide compound, may be prepared from the corresponding N-unsubstituted succinimide compound according to a procedure as described in Japanese Patent Publication (unexamined) No. 87262/1985 and EP—A—117569. Similarly, compound (III), i.e. the N-substituted piperazine, may be produced from the corresponding N-unsubstituted piperazine according to a procedure described in said literature.

The most common anti-anxiety drugs are benzodiazepine compounds, such as diazepam. These benzodiazepine compounds however, have a strong central nervous system depressing activity. For instance, they produce a great potentiation of hexobarbital anesthesia, which is an index of drowsiness and in fact, some benzodiazepine compounds are used as drugs inducing drowsiness. Such a depressing effect on the central nervous system is a side effect detracting from the use of the compounds as anti-anxiety drugs.

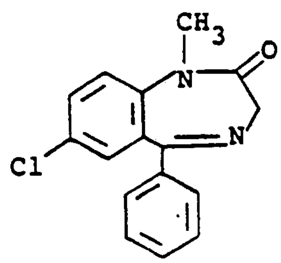

diazepam

Spiroimide compounds (e.g. buspirone) and succinimide compounds (e.g. SM—3997) as recently developed show a considerably reduced depressing effect on the central nervous system (such as potentiation of hexobarbital anesthesia) compared to benzodiazepine compounds. Therefore, they are useful as selective anti-anxiety drugs.

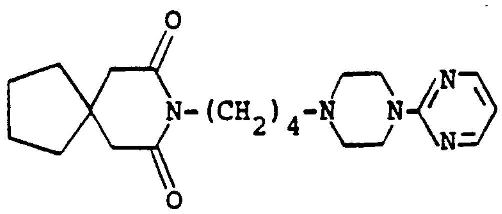

buspirone

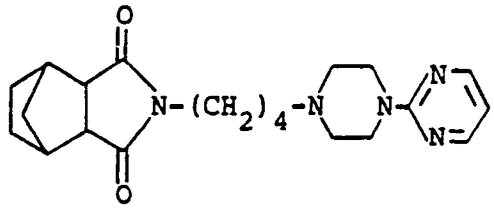

SM-3997

The succinimide derivates (I) of the invention have been found to show anxiolytic activity with a reduced depressing effect on the central nervous system. For they have an anti-anxiety effect with a weak drowsiness inducing effect. Therefore they can be used with patients showing anxiety symptoms or slight sleeplessness. These characteristic properties of the succinimide derivatives (I) are evidenced by the pharmacological test results set forth below.

Results of pharmacological test:
(1) Anti-anxiety activity

The anti-anxiety activity of the succinimide derivatives (I) was proved by the anti-conflict test, which was carried out according to the method described in Vogel et al.: Psychopharmacologia, *21*, 1 (1971)" with some modifications.

SD strain male rats (bodyweight 200 to 240 g) were forced to abstain from water for 24 hours before the test and placed into a measuring device. This device was designed to measure the frequency of rats drinking water inspite of receiving electric shocks: the number of licks and the frequency of the shocks received within 32 minutes were counted; 6 licks corresponded to drinking water for 1 second. Initially, no electric shocks were applied at all. Rats showing more than 300 licks were forced to abstain from water for another 24 hours and were then subjected to the test in which one electric shock (70 V, 0.35 mA) was applied per 20 licks. The number of times the rats drank water were counted. Rats, in which the frequency of drinking water was suppressed by the electric shock to 260 licks, were regarded as being in a conflict state and were subjected to the test.

Said rats were divided into several groups (8 to 10 rats per group), and the test compounds were intraperitoneally administered to them. One hour after administration, the rats were again placed into the measuring device and the frequency of electric shocks (70 V, 0.35 mA, 1 shock/20 licks) received while drinking water was counted.

Diazepam which is a known anti-anxiety drug was used as control compound. The results are shown in Table 1.

Table 1

| Compound | Dose (mg/kg) (i.p.) |
|---|---|
| N-[4-{4-(5-Fluoro-2-pyrimidinyl-1-piperazinyl}butyl]bicyclo[2.2.1]heptane-2,3-di-exo-carboximide (Compound No. 1) | 4 |
| N-[4-{4-(3-Chloro-2-pyridyl-1-piperazinyl}butyl]bicyclo[2.2.1]heptane-2,3-di-exo-carboximide (Compound No. 9) | 2 |
| Diazepam | 2 |

As is apparent from the above test results, the anti-conflict activity of the succinimide derivatives (I) is nearly equal to that of diazepam.

(2) Potentiating activity of hexobarbital anesthesia.

The test was carried out according to the method described by Ueki et al: Folia Farmacol. Japon, *77*, 483—509 (10981)", but somewhat modified.

The test compound was orally administered to dd strain male mice (bodyweight, 23 to 27 g). One hour later, hexobarbital (100 mg/kg) was intraperitoneally administered to said mice. The duration of anesthesia, as measured by loss of righting reflex, was recorded for each mouse. Where the hexobarbital anesthesia lasted for a period more than twice as long as the average time in the control group, anesthesia potentiation was considered positive (+).

The results are shown in Table 2.

Table 2

| Compound | Dose (mg/kg) (p.o.) | Judgement |
|---|---|---|
| Diazepam | 1 | + |
| Compound No. 1 | 100 | + |
| Compound No. 9 | 100 | + |
| SM-3997 | 100 | negative |
| Buspirone | 100 | negative |

According to the anti-climbing test in mice [Protais et al.: Psychopharmacology, *50*, 1—6 (1976)], some of the succinimide derivatives (I) including Compound Nos. 1 and 9 have been shown to possess significant anti-pyschotic activity. Accordingly, they may be used for treatment of pyschosis.

In general, the succinimide derivatives (I) exhibit central activity with weak peripheral side action. Their central activity is thus selective.

For therapeutic use, the succinimide derivatives (I) or their acid addition salts may be formulated into conventional pharmaceutical preparation forms suitable for oral or parenteral administration. Examples of such pharmaceutical preparation forms are tablets, capsules, solutions, etc. When being formulated, they are usually combined with suitable carriers or diluents such as fillers, binders or stabilizers.

The dosage of the succinimide derivatives (I) may vary from and also depend upon the degree of the symptom, age and bodyweight of the patient, route of administration, etc. In the case of oral administration, the compound can in general be administered to an adult in a daily amount of from about 0.2 to 1500 milligrams, preferably from about 1 to 500 milligrams, at once or in several doses.

The present invention will be further illustrated in detail by means of the following Reference Examples and Examples, which are not, however, intended to limit the scope of the invention.

Reference Example 1

A mixture of bicyclo[2.2.1]heptane-2,3-di-exo-carboximide (50 g), tetramethylene bromide (327 g), potassium carbonate (50 g) and acetone (500 ml) was heated under reflux for 5 hours. The mixture was cooled to room temperature, and insoluble materials were removed by filtration. The filtrate was concentrated by distillation under reduced pressure to give N-(4-bromobutyl)bicyclo[2.2.1]heptane-2,3-di-exo-carboximide (71.4 g) in the form of an oil.

Yield: 78.6%. B.P., 173—180°C/0.04 mmHg.

IR $\nu_{max}^{film}$ ($cm^{-1}$): 1765, 1700, 1430, 1395.

In the same manner as in Reference Example 1, the following compounds were obtained.

N-(4-Bromobutyl)bicyclo[2.2.1]heptane-2,3-di-endo-carboximide.

IR $\nu_{max}^{film}$ ($cm^{-1}$): 1765, 1700;

N-(4-Bromobutyl)bicyclo[2.2.2]octane-2,3-di-carboximide.

IR $\nu_{max}^{film}$ ($cm^{-1}$): 1765, 1690;

Reference Example 2

A solution of 2,3-chloropyridine (13.1 g) and anhydrous piperazine (37.9 g) in n-butanol (350 ml) was heated under reflux for 16 hours. The solvent was removed by evaporation under reduced pressure, and the residue was diluted with aqueous sodium hydroxide solution and extracted with dichloromethane. From the extract, the solvent was removed by distillation under reduced pressure to give 1-(3-chloro-2-pyridyl)piperazine (15.7 g) in the form of an oil.

IR $\nu_{max}^{film}$ ($cm^{-1}$): 3280, 1575.

M.P. 142—144°C (hydrochloride).

In the same manner as in Reference Example 2, the following compounds were obtained.
1-(5-Bromo-2-pyrimidinyl)piperazine,
M.P., 70—72°C;
1-(5-Fluoro-2-pyrimidinyl)piperazine,
M.P., 38.5—40°C;
1-(4-Methyl-2-pyrimidinyl)piperazine,
IR $\nu^{film}_{max}$ ($cm^{-1}$): 3180, 1560;
1-(5-Benzyloxy-2-pyrimidinyl)piperazine,
M.P., 95—96°C;
1-(4,6-Dimethyl-2-pyrimidinyl)piperazine,
M.P., 85—86°C;
1-(4,6-Dimethoxy-2-pyrimidinyl)piperazine,
M.P., 100—103°C;
1-(2-Amino-4-methyl-6-pyrimidinyl)piperazine,
M.P., 177—178°C;
1-(5-Chloro-2-pyridinyl)piperazine,
M.P., 59—61°C;
1-(3-Methoxy-2-pyridinyl)piperazine,
M.P., 50.5—51.5°C;
1-(3-Cyano-2-pyridinyl)piperazine,
M.P., 104—106°C.

Example 1

A mixture of N-(4-bromobutyl)bicyclo[2.2.1]heptane-2,3-di-exo-carboximide (1.8 g), 1-(5-fluoro-2-pyrimidinyl)piperazine (1.09 g), potassium carbonate (1.7 g) and N,N-dimethylformamide (20 ml) was stirred at 100 to 110°C for 4 hours. After completion of the reaction, the resultant mixture was poured into water and extracted with ethyl acetate. The oily residue was purified by silica gel column chromatography to give N-[4-{4-(5-fluoro-2-pyrimidinyl)-1-piperazinyl}butyl]bicyclo[2.2.1]heptane-2,3-di-exo-carboximide (Compound No. 1). M.P., 126—129°C.

In the same manner as in Example 1, the compounds shown in Table 3 were obtained.

Table 3

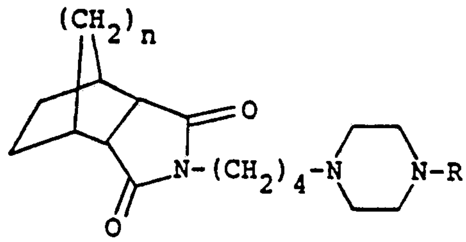

(I)

| Compound No. | R | n | exo or endo | M.P. (°C) |
|---|---|---|---|---|
| 2 | 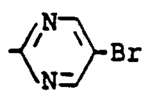 | 1 | exo | 112 - 114 |
| 3 | 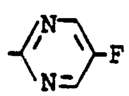 | 1 | endo | 239 - 240 (HCl) |
| 4 | 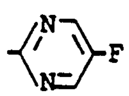 | 2 | - | 244 (dec.) (HCl) |
| 5 | 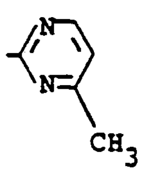 | 1 | exo | 103 - 104 |
| 6 | 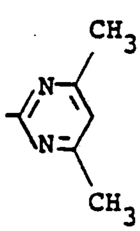 | 1 | exo | 248 - 250 (HCl) |

(Continued)

| Compound No. | R | n | exo or endo | M.P. (°C) |
|---|---|---|---|---|
| 7 | 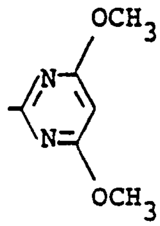 | 1 | exo | 105 - 107 |
| 8 | 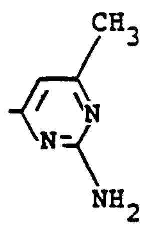 | 1 | exo | 189 - 191 |
| 9 | 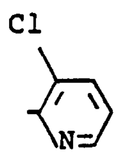 | 1 | exo | 196 - 200 (HCl) |
| 10 | 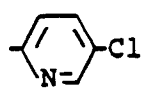 | 1 | exo | 121 - 122 |
| 11 | 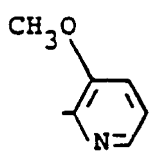 | 1 | exo | 196.5 - 198.5 (HCl) |
| 12 | 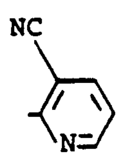 | 1 | exo | 180 - 184 (HCl) |
| 13 | 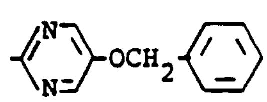 | 1 | exo | 116 - 119 |

Example 2

A mixture of N-[4-{4-(5-benzyloxy-2-pyrimidinyl)-1-piperazinyl}butyl]bicyclo[2.2.1]heptane-2,3-di-exo-carboximide (110 mg), 10% palladium-carbon (11 mg) and methanol (30 ml) was hydrogenated at 100°C for 2 hours under a pressure of 8 kg/$cm^2$. After completion of the reaction, the palladium catalyst was removed by filtration. The filtrate was evaporated under reduced pressure, and the residue was purified by silica gel

column chromatography using 10% methanol in chloroform as an eluent to give N-[4-{4-(5-hydroxy-2-pyrimidinyl)-1-piperazinyl}butyl]bicyclo[2.2.1]heptane-2,3-di-exo-carboximide (Compound No. 14). M.P., 202—203°C.

## Claims

1. A succinimide derivative of the formula:

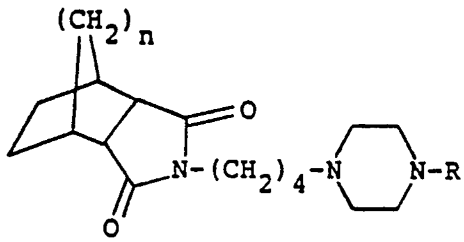

(I)

wherein R is a pyridyl or pyrimidinyl group each being substituted by at least one substituent selected from the group consisting of halogen, lower alkyl, lower alkoxy, cyano, benzyloxy, hydroxyl and amino and n is an integer of 1 or 2, or a pharmaceutically acceptable acid addition salt thereof.

2. A process for preparing the succinimide derivative or its pharmaceutically acceptable acid addition salt as claimed in claim 1, which comprises (a) reacting a compound of the formula:

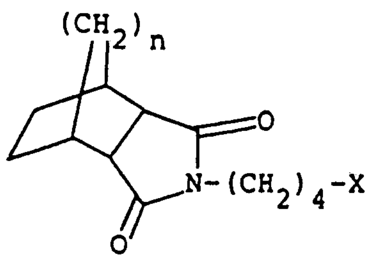

(II)

wherein n is as defined in claim 1 and X is a halogen atom with a compound of the formula:

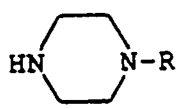

(III)

wherein R is as defined in claim 1 to give the succinimide derivative (I), or (b) subjecting a compound of the formula:

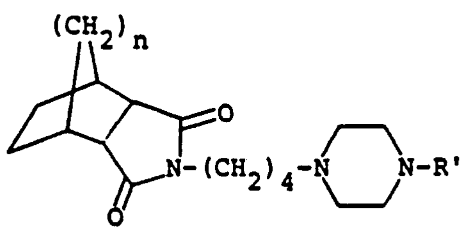

wherein R' is a pyridyl or pyrimidinyl group substituted with benzyloxy and n is as defined in claim 1 to reduction to give a compound of the formula:

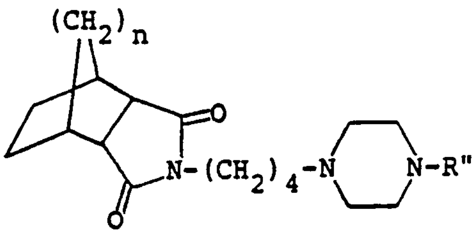

wherein R'' is a pyridyl or pyrimidinyl group substituted by hydroxyl and n is as defined in claim 1.

3. A pharmaceutical composition which comprises as an active ingredient a pharmaceutically effective amount of at least one of the succinimide derivative (I) or its pharmaceutically acceptable acid addition salt as claimed in claim 1 and at least one pharmaceutically acceptable carrier or diluent.

4. A pharmaceutical composition for treating anxiety, which comprises as an active ingredient a pharmaceutically effective amount of at least one of the succinimide derivative (I) or its pharmaceutically acceptable acid addition salt as claimed in claim 1 and at least one pharmaceutically acceptable carrier or diluent.

5. The use of the succinimide derivative (I) or its pharmaceutically acceptable acid addition salt as claimed in claim 1 for the preparation of an anti-anxiety drug.

**Patentansprüche**

1. Succinimidderivat der Formel

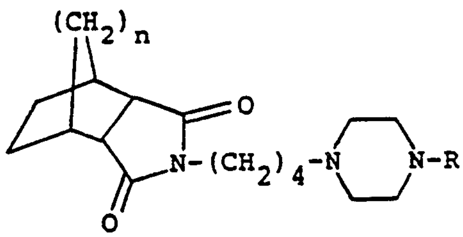

(I)

in der R eine Pyridyl- oder Pyrimidinylgruppe darstellt, die jeweils mit mindestens einem der Substituenten der Gruppe Halogenatom, Niederalkyl- oder Niederalkoxyrest, Cyano-, Benzyloxy-, Hydroxy- oder Aminogruppe substituiert ist, und n eine ganze Zahl mit einem Wert von 1 oder 2 bedeutet, oder einem pharmazeutisch verträglichen Säureadditionssalz davon.

2. Verfahren zur Herstellung der Succinimidderivate oder der phamazeutisch verträglichen Säureadditioanssalze davon nach Anspruch 1, dadurch gekennzeichnet, daß man (a) eine Verbindung der Formel

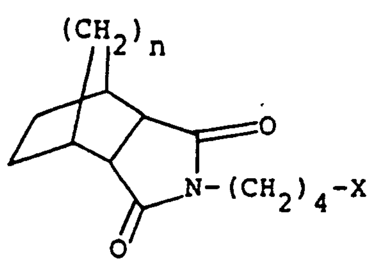

(II)

in der n die in Anspruch 1 angegebene Bedeutung hat, und X ein Halogenatom darstellt mit einer Verbindung der Formel

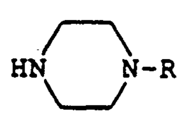

(III)

in der R die in Anspruch 1 angegebene Bedeutung hat zum Succinimidderivat (I) umsetzt oder (b) eine Verbindung der Formel

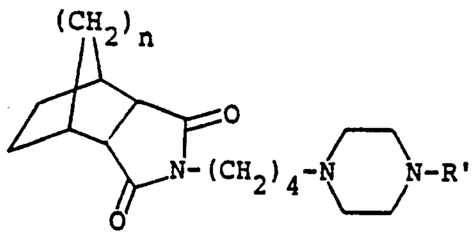

in der R' eine Pyridyl- oder Pyrimidinylgruppe, die mit einer Benzyloxygruppe substituiert ist, darsellt und n die in Anspruch 1 angegebene Bedeutung hat, zu einer Verbindung der Formel

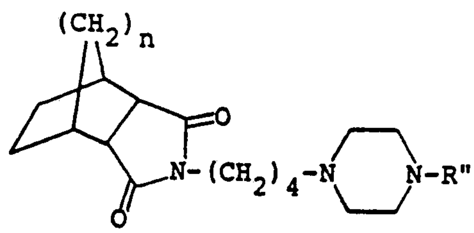

reduziert, in der R'' eine Pyridyl- oder Pyrimidinylgruppe, die mit einer Hydroxygruppe substituiert ist, darstellt und n die in Anspruch 1 angegebene Bedeutung hat.

3. Arzneimittel, enthaltend als Wirkstoff eine pharmazeutisch wirksame Menge von mindestens einem der Succinimidderivate (I) oder dessen pharmazeutisch verträglichem Säureadditionssalz nach Anspruch 1 und mindestens einem pharmazeutisch verträglichen Träger oder Verdünnungsmittel.

4. Arzneimittel zur Behandlung von Angst, enthaltend als Wirkstoff eine phamazeutisch wirksame Menge von mindestens einer der Succinimiderivate (I) oder dessen pharmazeutisch verträgliche Säureadditionssalze nach Anspruch 1 und mindestens einem pharmazeutisch verträglichen Träger oder Verdünnungsmittel.

5. Verwendung der Succinimidderivate (I) oder dessen pharmazeutisch verträgliche Säureadditionssalze nach Anspruch 1 zur Herstellung von einem Anxiolytikum.

**Revendications**

1. Un dérivé de succinimide de formule

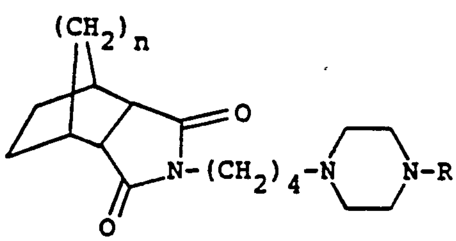

(I)

dans laquelle R est un groupe pyridyle ou pyrimidinyle substitués chacun par au moins un substituant choisi parmi halogène, alkyle inférieur, alcoxy inférieur, cyano, benzoxy, hydroxyle et amino et n est égal à 1 ou 2, ou un de ses sels d'addition d'acides pharmaceutiquement acceptables.

2. Un procédé pour fabriquer le dérivé de succinimide ou son sel d'addition d'acide pharmaceutiquement acceptable selon la revendication 1, qui comprend (a) la réaction d'un composé de formule

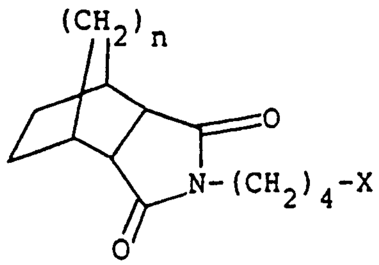

(II)

dans laquelle n est tel que défini à la revendication 1 et X est un atome d'halogène avec un composé de formule

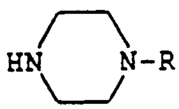

(III)

dans laquelle R est tel que défini à la revendication 1 pour donner le dérivé de succinimide (I), ou (b) la réduction d'un composé de formule

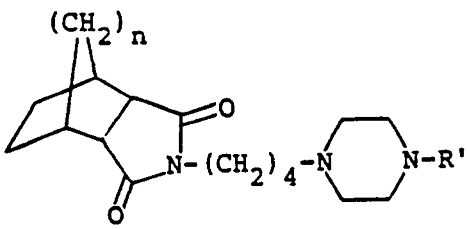

dans laquelle R' est un groupe pyridyle ou pyrimidinyle substitué par benzoxy et n est tel que défini à la revendication 1 pour donner un composé de formule

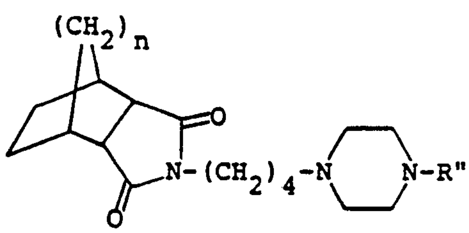

dans laquelle R'' est un groupe pyridyle ou pyrimidinyle substitué par hydroxy et n est tel que défini à la revendication 1.

3. Une composition pharmaceutique qui comprend comme ingrédient actif une quantité pharmaceutiquement efficace d'au moins un dérivé de succinimide (I) ou son sel d'addition d'acide pharmaceutiquement acceptable selon la revendiation 1 et au moins un support ou diluant pharmaceutiquement acceptable.

4. Une composition pharmaceutique pour traiter l'anxiété, qui comprend comme ingrédient actif une quantité pharmaceutiquement efficace d'au moins un dérivé de succinimide (I) ou son sel d'addition d'acide pharmaceutiquement acceptable selon la revendication 1 et au moins un support ou diluant pharmaceutiquement acceptable.

5. L'utilisation du dérivé de succinimide (I) ou de son sel d'addition d'acide pharmaceutiquement acceptable selon la revendication 1 pour la fabrication d'un médicament anti-anxiété.